# EUROPEAN PATENT SPECIFICATION

(11) **EP 3 081 208 B1**
(45) Date of publication and mention of the grant of the patent: **21.08.2019**
(21) Application number: 15001047.8
(22) Date of filing: 13.04.2015
(51) Int. Cl.: A61K 8/73, A61Q 17/04, A61K 8/04, A61K 8/06

(54) **SKIN CARE SPRAY COMPOSITIONS COMPRISING MICROFIBRILLATED CELLULOSE**
HAUTPFLEGESPRAYZUSAMMENSETZUNGEN MIT MIKROFIBRILLIERTER CELLULOSE
COMPOSITIONS DE PULVÉRISATION POUR SOINS DE LA PEAU CONTENANT DE LA CELLULOSE MICROFIBRILLÉE

(43) Date of publication of application: 19.10.2016
(73) Proprietor: Borregaard AS, 1721 Sarpsborg (NO)
(72) Inventor: BLELL, Rebecca, 1701 Sarpsborg (NO); NYGÅRD VOLD, Inger Mari, 1701 Sarpsborg (NO); ØVREBRØ, Hans Henrik, 1701 Sarpsborg (NO); GONERA, Antje, 1540 Vestby (NO)
(74) Representative: Ricker, Mathias

(56) References cited:
- EP-A1- 2 526 922
- EP-A1- 2 929 874
- WO-A2-00/47628
- WO-A2-2004/026263
- FR-A1- 2 794 466
- US-A- 4 500 546
- US-A1- 2008 146 485
- US-A1- 2010 009 891

## Description

The present invention relates to the use of microfibrillated cellulose as an anti-dripping agent in a sprayable skin care composition, in particular a sunscreen spray composition. The present invention also relates to a sprayable skin care composition comprising at least one microfibrillated cellulose and a method for preparing the same.

### Background of the Invention

Due to the increasing awareness of the skin damaging effects of UV radiation, there is a growing demand for sunscreen compositions. In particular, sunscreen sprays of comparatively low viscosity are getting increasingly popular due to the easy application, even skin coverage and the comfortable skin-feel. However, these sunscreen sprays have the undesirable tendency to drip or run after application, which may result in the contamination of sensitive regions of the body, such as the eyes, waste of sunscreen composition, as well as pollution of clothes. It is thus desirable to make the application of such sunscreen sprays easier to control, in particular, to avoid dripping of the sunscreen composition upon application to the skin.

This is commonly achieved by addition of synthetic polymers to the sunscreen sprays. However, synthetic polymers do not satisfy increasing demands for ingredients of natural origin in personal care products. Therefore, it is an object of the present invention to provide a new type of sprayability-improving agent which is of natural origin.

Cellulose-based materials are used in personal care products, in principle, usually as a swellable media or as suspending aids cosmetic formulations.

US 2004/0156811A1 describes microcrystalline cellulose as an enhancing agent for wrinkle-hiding in decorative skin cosmetics.

Microfibrillar cellulose has been used in personal care products (e.g. body washes, hand soaps and conditioners) mostly as a rheology modifier (as a thickener, a suspending aid and/or as a gel forming agent). WO2013031030A1 describes MFC as a gel forming agent in cosmetic sheets. WO2013094077A1 describes microfibrillar cellulose as building material for cosmetic sheets. EP2307100A2 describes MFC as a rheology modifier in liquid cleanser compositions.

EP1144103A2 describes nanofibrils of amorphous cellulose as an emulsifying agent and/or a dispersion stabilizer. HU9903102A2 describes nanofibrils of amorphous cellulose as a viscosity modifier in cosmetics and detergent products.

### Summary of the Invention

The above mentioned object and other objects are achieved by the teaching of the present invention. Surprisingly, it has been found that the addition of microfibrillated cellulose (MFC) to cosmetic compositions efficiently prevents dripping and running of the composition upon application while maintaining good sprayability.

Spray patterns of sunscreen formulations with and without 0.3% microfribrillated cellulose were analyzed. The MFC containing formulation had the desired viscous consistency (a thick emulsion) but was nevertheless still sprayable. Without wishing to be bound by theory, it is believed that good sprayability is caused by the thixotropic properties / the shear thinning effect of MFC, meaning that MFC dispersions are relatively viscous under static conditions but will flow (become thin, less viscous) when shaken, agitated, or otherwise stressed, i.e. the viscosity of an MFC dispersion is reduced when shear forces are applied. As a consequence, zero shear viscosity, η₀, is comparatively high, while the viscosity at high shear is comparatively reduced. This results, among others, in the microfibrillated cellulose having a good ability to stabilize dispersions in a stationary state, while, at the same time, rendering the dispersion easier to process, for example in regard to spraying.

Upon application by spraying, the MFC containing formulation did not drop or drip - the spray pattern remained stable and unchanged. Without wishing to be bound by theory, it is believed that the stable spray pattern is due to the comparatively high zero shear viscosity of the microfibrillated cellulose. In comparison, the control sunscreen formulation without MFC was a thin liquid emulsion and dripped upon spraying. Thus, MFC allows for spraying of a comparatively viscous formulation and efficiently prevents dripping upon application by spraying.

Thus, in a first aspect, the present invention relates to the use of microfibrillated cellulose (MFC) as an anti-dripping agent in a sprayable skin care composition,wherein the MFC is unmodified MFC or physically modified by physical adsorption of at least one amphiphilic compound,wherein the concentration of the MFC in the sprayable skin care composition is from 0.012% to 0.6% by weight, and wherein the MFC originates from wood, annual plants, cotton, flax, straw, ramie, sugar cane bagasse, certain algae, jute, sugar beet, citrus fruits, waste from the food processing industry or energy crops.

Preferably, the microfibrillated cellulose results in a dispersion having a viscosity, η₀, of at least 100 Pa*s, preferably at least 1000 Pa*s, further preferably at least 4000 Pa*s, even further preferably at least 5000 Pa*s, as measured in water as solvent, at a shear rate of 0.0001 s⁻¹ and at a solids content of the MFC of 0.50 wt%.

Preferably, the microfibrillated cellulose results in a dispersion having a zero shear viscosity, η₀, of at least 5000 Pa*s, preferably at least 6000 Pa*s, further preferably at least 7000 Pa*s, as measured in polyethylene glycol (PEG) as solvent, and at a solids content of the MFC of 0.65%. Preferably, the microfibrillated cellulose has comparatively high shear thinning effects and good stabilizing effects on an MFC containing dispersion at stationary state, meaning that upon application, e.g. by spraying, the MFC has the ability to keep the various components of the dispersion together thereby stabilizing the spray pattern.

### Detailed Description of the Invention

In the following, the invention is described with reference to the enclosed figure, wherein:

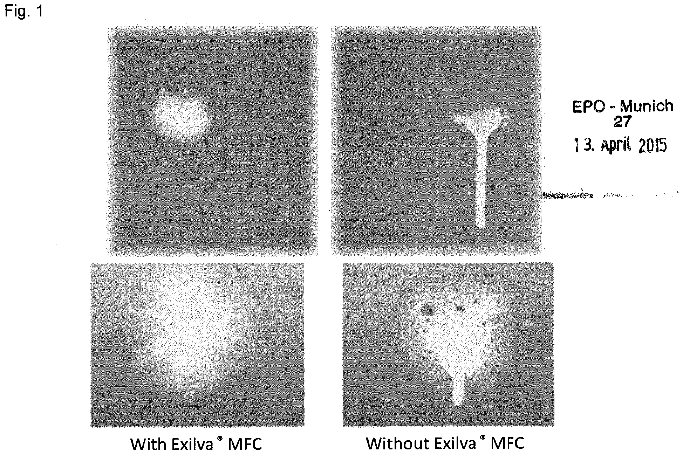

**Figure 1** shows the spray pattern of cosmetic formulations with and without MFC. MFC efficiently prevents dripping of the cosmetic formulation. In comparison, the cosmetic formulation without MFC shows pronounced running and dripping.

Advantages, preferred embodiments and possible applications of the present invention are described in the following.

Microfibrillated cellulose (MFC) as used within the claimed composition and in the meaning of the present invention relates to cellulose fibers of any conceivable origin. In particular, MFC according to the present invention relates to cellulose in fiber form that has been subjected to a mechanical treatment in order to increase the fibers' specific surface and to reduce their size in terms of cross-section and of length, wherein said size reduction leads to a fiber diameter in the nanometer range and a fiber length in the micrometer range.

MFC (also known as "microfibrillar cellulose", "reticulated cellulose", "superfine cellulose" or as "cellulose nanofibrils", among others) is prepared from cellulose fibers which are defibrillated using high pressure and/or high mechanical force. In the cellulose starting material (typically present as a "cellulose pulp") no, or at least not a significant or not even a noticeable portion of individualized and "separated" cellulose fibrils can be found. By contrast, in MFC, individual fibrils and fibril bundles can be easily discerned by way of conventional optical microscopy. Sometimes, these fibrils and bundles of fibrils are also described as "(micro)fibrils". In accordance with the present invention, any reference to "fibrils" also includes bundles of such fibrils. Microfibrillated cellulose is described e.g. in US 4 481 077, US 4 374 702 and US 4 341 807. According to US 4 374 702 ("Turbak"), microfibrillated cellulose has properties distinguishable from previously known celluloses.

Due to its large surface area and a high aspect ratio (length to width ratio), microfibrillated cellulose is believed to have a good ability to form rigid networks. In solution, MFC typically forms a highly viscous gel-like dispersion with shear thinning properties. The large surface area of the MFC and the high amount of accessible hydroxyl groups cause MFC to typically have a high water holding capacity.

MFC in accordance with "Turbak" is produced by passing a liquid suspension of cellulose through a small diameter orifice in which the suspension is subjected to a large pressure drop and a high velocity shearing action followed by a high velocity decelerating impact and repeating the passage of said suspension through the orifice until the cellulose suspension becomes a substantially stable suspension. The process converts the cellulose into microfibrillated cellulose without substantial chemical change of the cellulose starting material.

An improved process for obtaining particularly homogeneous MFC is described in WO 2007/091942.

The microfibrillated cellulose in accordance with the present invention may be produced according to any process known in the art. Preferably, said method comprises at least one mechanical step and at least one homogenizing step. The mechanical pretreatment step preferably is or comprises a refining step. The method can also comprise a chemical pretreatment step. One example of such pretreatment step might be oxidation of the hydroxyl groups on the surface of the microfibrils to carboxylic acids. Negative charges of the carboxylic groups cause repulsion between the microfibrils which aids the defibrillation of the cellulose.

The purpose of the mechanical pretreatment step is to "beat" the cellulose pulp in order to increase the accessibility of the cell walls, i.e. increase the surface area and therefore to increase the water retention value. In the refiner that is preferably used in the mechanical pretreatment step, one or two rotating disk(s) is/are employed, i.e. the cellulose pulp slurry is subjected to shear forces.

Prior to the mechanical or chemical pretreatment step, or in between the mechanical or chemical pretreatment steps or as the mechanical pretreatment step, enzymatic (pre)treatment of the cellulose pulp is an optional additional step that may be preferred for some applications. In regard to enzymatic pretreatment in conjunction with microfibrillating cellulose, the respective content of WO 2007/091942 is incorporated herein by reference.

The microfibrillated cellulose preferably has a high aspect ratio which corresponds to the ratio of the length of the microfibril bundle to its diameter (L/D). To this end, the pulp slurry is preferably passed through a homogenizer (a high-pressure homogenizer or a low-pressure homogenizer) and subjected to a pressure drop by forcing the pulp slurry between opposing surfaces, preferably orifices. The term "orifice" means an opening or a nozzle or a valve contained in a homogenizer suitable for homogenizing cellulose.

In particular embodiments, the length and/or the diameter of the cellulose fibrils and fibril bundles of the microfibrillated cellulose are reduced vis-à-vis the respective length and diameter of the cellulose fibers and fiber bundles making up the cellulose that was used as a starting point.

In particular embodiments, the average MFC fibril length is from 100 nm to 50 µm, preferably from 500 nm to 25 µm, more preferably from 1 µm to 10 µm, most preferably from 3 µm to 10 µm.

In particular embodiments, the average MFC fibril diameter is from 1 nm to 500 nm, preferably from 5 nm to 100 nm, more preferably from 10 nm to 50 nm, most preferably from 10 nm to 30 nm.

In particular embodiments, the average MFC fibril aspect ratio is comparatively high.

Fibril length and fibril diameter are determined by imaging using Atomic Force Microscopy and/or Scanning Electron Microscopy.

In particular embodiments, the specific surface area of the MFC is comparatively high. Within the context of the present application, the term "specific surface area" refers to the total surface area of an MFC material per unit of mass.

In particular embodiments, the microfibrillated cellulose has a water holding capacity (water retention capacity) of at least 75, preferably at least 80, further preferably at least 100. The water holding capacity describes the ability of the MFC to retain water within the MFC structure and this again relates to the accessible surface area. According to the invention, the water holding capacity is measured by diluting an MFC sample to a 0.3% solids content in water and then centrifuging the sample at 1000 G for 15 minutes. The clear water phase is separated from the sediment and the sediment is weighed. The water holding capacity is given as (mV/mT)-1 where mV is the weight of the wet sediments and mT is the weight of dry MFC analyzed.

In particular embodiments, the microfibrillated cellulose results in gel-like dispersion having a zero shear viscosity, η₀, of at least 5000 Pa*s, preferably at least 6000 Pa*s, further preferably at least 7000 Pa*s, as measured in polyethylene glycol (PEG) as solvent, and at a solids content of the MFC of 0.65%.

Preferably, the microfibrillated cellulose results in a dispersion having a viscosity, η₀, of at least 100 Pa*s, preferably at least 1000 Pa*s, further preferably at least 4000 Pa*s, even further preferably at least 5000 Pa*s, as measured in water as solvent, at a shear rate of 0.0001 s⁻¹ and at a solids content of the MFC of 0.50 wt%.

The zero shear viscosity, η₀ ("*viscosity at rest*") is a measure for the stability of the three-dimensional network making up the gel-like dispersion. The "*zero shear viscosity*" as disclosed and claimed herein is measured on a rheometer of the type *Anton Paar* Physica MCR 301 with PEG 400 as the solvent. "PEG 400" is a polyethylene glycol with a molecular weight between 380 and 420 g/mol and is widely used in pharmaceutical applications and therefore commonly known and available. The temperature during measurements is 25°C and a "plate-plate" geometry is used (diameter: 50 mm).

The MFC used in this invention preferably has improved viscous properties. Without wishing to be bound by theory, it is believed that the three-dimensional networks of an MFC dispersion are stabilized, while these stabilized networks may be easily broken apart, once a shear force is applied, for example when the gel is to be transported (pumped or sprayed) or the like. Due to the high zero shear viscosity, as soon as the sprayed material hits the surface it is sprayed on, the gel structure of the dispersion is reformed (i.e. the viscosity increases) and no dripping occurs.

In principle, the raw material for the cellulose microfibrils may be any cellulosic material, in particular cellulose originating from wood, annual plants, cotton, flax, straw, ramie, sugar cane bagasse, certain algae, jute, sugar beet, citrus fruits, waste from the food processing industry or energy crops or cellulose of bacterial or animal origin, e.g. from tunicates.

In particular embodiments, wood-based materials are used as raw materials, either hardwood or softwood or both (in mixtures). Further preferably softwood is used as a raw material, either one kind or mixtures of different soft wood types. The term MFC, in accordance with the present invention, encompasses a single kind of microfibrillated cellulose as well as a mixture of structurally different microfibrillated celluloses, such as a mixture of unmodified and physically modified microfibrillated cellulose. The microfibrillated cellulose in accordance with the present invention may be unmodified with respect to its functional group or may be physically modified. In one embodiment, the MFC, in accordance with the present invention, is substantially free of cationic substituents. In particular embodiments, the MFC is unmodified MFC.

The cellulose microfibrils may also be modified by a substantially 'physical' method, which does not involve the formation of chemical bonds to a substantial extend, in particular, either by adsorption at the surface, or by spraying, coating or encapsulation of the microfibril. Preferred modified microfibrils can be obtained by physical adsorption of at least one compound. The microfibrils can be modified by physical adsorption of at least one amphiphilic compound (surfactant).

In one embodiment, microfibrils are modified by physical adsorption of at least one non-ionic surfactant. EP2 408 857 describes processes of preparing surface modified MFC. The physical modification of the MFC surface may take place before or after the defibrillation step.

Physical modification of the cellulose microfibril surface may occur prior to the mechanical pretreatment step, between the mechanical pretreatment step and the defibrillation step or after the defibrillation step.

The microfibrillated cellulose (MFC) according to the present invention may be subjected to at least one dewatering and/or drying step. The at least one drying step is preferably selected from freeze-, spray- or roller-drying, drying in a convection oven, flash drying or the like. 'Never-dried' microfibrillated cellulose may also be used and the microfibrillated cellulose used in the present invention might have a dry content ranging from 0.1%-100% before it is added to a skin care composition.

In particular embodiments, the dry content of the MFC is from 0.1% to 100%, preferably from 0.2% to 40%, more preferably from 0.5% to 25%, even more preferably from 1% to 20%, before the MFC is added to the composition.

Within the context of the present application, the term "anti-dripping agent" refers to a substance able to prevent a liquid composition from trickling or running down a vertical surface that has been sprayed with the liquid composition to at least some extent. For example, the distance of travel in a given time interval upon application to a vertical surface by spraying may be reduced by more than 5%, 10%, 20%, 30%, 40%, 50%, 60%; 70%, 80%, 90%, 95% or more in comparison to the distance of travel of a corresponding liquid composition not containing the anti-dripping agent. For example, a 'dripping reducing' effect exists, if the distance of travel during the first 10 seconds upon application to a vertical cardboard surface by spraying is reduced by at least 10% in comparison to the distance of travel of a corresponding liquid composition not containing the anti-dripping agent. The distance of travel represents the length of the longest protrusion of liquid composition extending from the original spray pattern obtained immediately upon application, measured from the bottom of the extrusion (i.e. from the outer boundary of the original spray pattern) to its top. According to one embodiment, the microfibrillated cellulose is provided in a sprayable skin care composition, particularly a sunscreen spray composition, an after-sun spray composition, a face spray composition, a body spray composition, an insect repellent spray composition, a tanning spray composition, a deodorant spray composition, a toner spray composition, a moisturizing spray composition, a disinfection spray composition, a swelling relief spray composition, a wound care spray composition, an anti-allergen spray composition, a scar treatment composition, an antimicotic spray composition and an antibacterial and/or antiviral dermatological spray composition.

In particular embodiments, the skin care composition further comprises at least one UV filter.

Within the context of the present application, the term "UV filter" refers to an agent able to block, absorb and/or reflect UV rays. Suitable UV filters may be selected from, but are not limited to, zinc oxide, titanium dioxide, Avobenzone, Tinosorb S, Tinosorb M, Mexoryl SX, Mexoryl XL, Helioplex, Octinoxate, Octocrylene, Oxybenzone, Octisalate, Homosalate, Uvinul T 150, Cinoxate, aminobenzoic acid, Padimate O, Ensulizole, dioxybenzone, Meradimate, Sulisobenzone, Trolamine salicylate, Enzacamene, Bisdisulizole Disodium, Uvinul A Plus, Uvasorb HEB, Parsol SLX and Amiloxate.

The skin care composition may include further components or ingredients which are compatible with human skin. The ingredients may be chosen according to the selected application of the product and may be chosen from, but are not limited to, excipients, surfactants, emulsifiers, thickeners, vitamins, fats, oils, waxes, humectants, UV filters, preservatives, water, alcohol, perfumes, antifoaming agents, foam stabilizers, polymers, electrolytes, organic solvents, silicone derivatives and coloring agents.

The formulation of pharmaceutically-acceptable dermatological and cosmetic preparations, in principle, is known in the art.

In particular embodiments, the concentration of the microfibrillated cellulose in the skin care composition is from 0.012% to 7.5% by weight, preferably from 0.03% to 3% by weight, more preferably from 0.06% to 1.5% by weight, most preferably from 0.15% to 0.6% by weight.

In a second aspect, the present invention relates to a sprayable skin care composition comprising at least one microfibrillated cellulose, said at least one microfibrillated cellulose resulting in a dispersion having a zero shear viscosity, η₀, of at least 5000 Pa*s, preferably at least 6000 Pa*s, further preferably at least 7000 Pa*s, as measured in polyethylene glycol (PEG) as solvent, and at a solids content of the MFC of 0.65%.

In particular embodiments, the sprayable skin care composition is an oil in water emulsion. In particular embodiments, the sprayable skin care composition further comprises at least one UV filter.

In a third aspect, the present invention relates to a method of preparing a sprayable skin care composition comprising mixing at least one fatty phase and at least one aqueous phase thereby forming an oil in water (O/W) emulsion, said at least one aqueous phase comprising at least one microfibrillated cellulose, said at least one microfibrillated cellulose being characterized by resulting in a dispersion having a zero shear viscosity, η₀, of at least 5000 Pa*s, preferably at least 6000 Pa*s, further preferably at least 7000 Pa*s, as measured in polyethylene glycol (PEG) as solvent, and at a solids content of the MFC of 0.65%.

In particular embodiments, the method of preparing a sprayable skin care composition comprises mixing at least one UV filter and non-soluble particles with said at least one microfibrillated cellulose.

### Examples

### Analyzing spray patterns of sunscreen sprays with and without MFC

Spray patterns of sunscreen formulations with and without MFC were compared.

### Preparation of the sunscreen formulation

The microfibrillated cellulose used was characterized by resulting in a dispersion having a viscosity, η₀, of 5000 Pa*s, as measured in water as solvent, at a shear rate of 0.0001 s⁻¹ and at a solids content of the MFC of 0.50 wt%.

**Table 1. The sunscreen or sunspray formulations used in these tests were oil in water (O/W) emulsions containing the ingredients listed in the table below.**

| Pos. | Raw Material | INCI (Eu) | Supplier | % [w/w] 726/003 /001 | % [w/w] 726/003 /003 |
|---|---|---|---|---|---|
| **Phase A** | | | | | |
| 1 | Eumulgin B 2 Gesch. | CETEARETH-20 | BTC Europe GmbH | 0.500 | 0.500 |
| 2 | Lanette 16 | CETYL ALCOHOL | BTC Europe Gmbh | 1.000 | 1.000 |
| 3 | Tegosoft TN | C12-15 ALKYL BENZOATE | Evonik | 12.000 | 12.000 |
| 4 | Cetiol CC | DICAPRYLYL CARBONATE | BTC Europe Gmbh | 5.000 | 5.000 |
| 5 | Eusolex 9020 (105844) | BUTYL METHOXYDIBENZOY LMETHANE | Merck | 3.000 | 3.000 |
| 6 | Eusolex OCR (105377) | OCTORYLENE | Merck | 5.000 | 5.000 |
| 7 | Tinosorb S | BIS-ETHYLHEXYLOXYPH ENOL METHOXYPHENYL TRIAZINE | BTC Europe Gmbh | 1.000 | 1.000 |
| 8 | Uvinul T 150 | ETHYLXEXYL TRIAZONE | BTC Europe Gmbh | 1.000 | 1.000 |
| 9 | Euxyl PE 9010 | PHENOXYETHANOL, ETHYLHEXYLGLYCE RIN | Schülke & Mayr | 1.000 | 1.000 |

| **Phase B** | | | | | |
|---|---|---|---|---|---|
| 10 | Water, demin. | AQUA | | 53.850 | 50.850 |
| 11 | Edeta BD | DISODIUM EDTA | BTC Europe GmbH | 0.050 | 0.050 |
| 12 | Glycerin Ph. Eur. 85% | GLYCERIN, AQUA | Azelis | 5.000 | 5.000 |
| 13 | Pemulen TR-1 | ACRYLATES/C10-30 ALKYL ACRYLATE CROSS POLYMER | Gattefosse | 0.200 | 0.200 |
| 14 | MFC-Paste m/Phenoxyeth anol 10% aktiv. | | Borregaard AS | 0.000 | 3.000 |

| **Phase C** | | | | | |
|---|---|---|---|---|---|
| | | | | | |
| 15 | Water, demin. | AQUA | | 3.000 | 3.000 |
| 16 | Neo Heliopan Hydro (103089) | PHENYLBENZIMIDAZ OLE SULFONIC ACID | Symrise | 1.000 | 1.000 |
| 17 | NaOH (10% aq.) | AQUA, SODIUM HYDROXIDE | | 2.400 | 2.400 |

| **Phase D** | | | | | |
|---|---|---|---|---|---|
| 18 | Ethanol 96%ig, Sorte 1611v | ALCOHOL DENAT., AQUA | AHK Alkohol | 5.000 | 5.000 |
| | | | | 100,000 | 100,000 |

### Oil in Water (O/W) sun sprays

The preparation of the tested cosmetic formulations using the ingredients listed in table 2 is described in the following.

### Step 1: Preparation of the phases

Phase A: Pos. 1-9 were molten at approximately 80°C.
Phase B: Pos.13 resp. 14 was dispersed homogeneously in pos. 10-12 at room temperature with stirring (dissolver).
   The phase was heated to 80°C.
Phase C: Pos. 16 was dissolved at room temperature in pos. 15 by successive adding of pos.19 with stirring.
Phase D: Pos. 18 (ethanol) was provided separately.

### Step 2: Emulsifying process

- The hot aqueous phase B was added to the hot fatty phase A portion by portion with stirring (dissolver).
- Phase C was added with stirring (dissolver).
- The emulsion was cooled to approximately 40°C with stirring.
- Phase D was added with stirring.
- The emulsion was cooled to approximately 25°C and was homogenised by passing once over a Homozenta emulsifying machine (Fa. Zehnder, Switzerland) at stage 2.
- Finally the emulsion was degassed under vacuum.

The formulation work was carried out in laboratories of Institute Dr. Schrader, Max-Planck-Straße 6, D-37603 Holzminden, Germany.

### Results

The sunscreen formulation containing MFC showed no dripping upon application by spraying compared to the control formulation which upon spraying dripped down and did not remain where it has been sprayed. The respective spray patters are depicted in Fig. 1.

Table 2 shows the distance that the respective formulations ran down in a given time interval upon application by spraying to a vertical surface. The data summarized in table 2 clearly demonstrate the efficiency of MFC as an anti-dripping agent.

**Table 2. The vertical distance travelled in a given time for a formulation after spraying on a vertical substrate.**

| | Vertical distance of travel from sprayed area after spraying (cm) | Time of travel (s) |
|---|---|---|
| Control Sunscreen formulation (without MFC) | 6 | 10 |
| Sunscreen formulation with MFC | 0 | 60 |

Table 2 and Figure 1 show that the formulation with MFC does not move vertically (run down / drip) after spraying even after 60 seconds. The formulation with no MFC moves down the sprayed surface for around 6 cm in 10 seconds. The MFC therefore provides a non-dripping formulation.

## Claims

1. Use of microfibrillated cellulose (MFC) as an anti-dripping agent in a sprayable skin care composition,
wherein the MFC is unmodified MFC or physically modified by physical adsorption of at least one amphiphilic compound,
wherein the concentration of the MFC in the sprayable skin care composition is from 0.012% to 0.6% by weight, and
wherein the MFC originates from wood, annual plants, cotton, flax, straw, ramie, sugar cane bagasse, certain algae, jute, sugar beet, citrus fruits, waste from the food processing industry or energy crops.

2. The use according to claim 1, wherein the sprayable skin care composition is a sunscreen spray composition, an after-sun spray composition, a face spray composition, a body spray composition, an insect repellent spray composition, a tanning spray composition, a deodorant spray composition, a toner spray composition, a moisturizing spray composition, a disinfection spray composition, a swelling relief spray composition, a wound care spray composition, an anti-allergen spray composition, a scar treatment composition, an antimicotic spray composition and an antibacterial and/or antiviral dermatological spray composition.

3. The use according to any one of the preceding claims, wherein the average MFC fibril length is from 100 nm to 50 µm, preferably from 500 nm to 25 µm, more preferably from 1 µm to 10 µm, most preferably from 3 µm to 10 µm.

4. The use according to any one of the preceding claims, wherein the average MFC fibril diameter is from 1 nm to 500 nm, preferably from 5 nm to 100 nm, more preferably from 10 nm to 50 nm, most preferably from 10 nm to 30 nm.

5. The use according to any one of the preceding claims, wherein the microfibrillated cellulose results in a dispersion having a zero shear viscosity, η₀, of at least 5000 Pa*s, preferably at least 6000 Pa*s, further preferably at least 7000 Pa*s, as measured in polyethylene glycol (PEG) as solvent, and at a solids content of the MFC of 0.65% at a temperature of 25°C.

6. The use according to any one of the preceding claims, wherein the water holding capacity of the MFC is at least 75, preferably at least 80, more preferably at least 100.

7. The use according to any one of the preceding claims, wherein the MFC is physically modified by physical adsorption of at least one amphiphilic compound which is a non-ionic amphiphilic compound.

8. The use according to any one of the preceding claims, wherein the MFC is substantially free of cationic substituents.

9. The use according to any one of the preceding claims, wherein the dry content of the MFC is from 0.1% to 100%, preferably from 0.2% to 40%, more preferably from 0.5% to 25%, more preferably from 1% to 20%, before the MFC is added to the composition.

10. The use according to any one of the preceding claims, wherein the composition further comprises at least one UV filter.

## Patentansprüche

1. Verwendung von mikrofibrillierter Cellulose (MFC) als Antitropfmittel in einer sprühbaren Hautpflegezusammensetzung,
wobei die MFC unmodifizierte MFC ist oder durch physikalische Adsorption von mindestens einer amphiphilen Verbindung physikalisch modifiziert ist,
wobei die Konzentration der MFC in der sprühbaren Hautpflegezusammensetzung 0,012 Gew.-% bis 0,6 Gew.-% beträgt, und
wobei die MFC von Holz, einjährigen Pflanzen, Baumwolle, Flachs, Stroh, Ramie, Zuckerrohrbagasse, bestimmten Algen, Jute, Zuckerrüben, Zitrusfrüchten, Abfällen aus der lebensmittelverarbeitenden Industrie oder Energiepflanzen stammt.

2. Verwendung nach Anspruch 1, wobei die sprühbare Hautpflegezusammensetzung eine Sonnenschutzsprayzusammensetzung, eine After-Sun-Sprayzusammensetzung, eine Gesichtssprayzusammensetzung, eine Körpersprayzusammensetzung, eine Insektenschutzmittelsprayzusammensetzung, eine Bräunungssprayzusammensetzung, ein Deodorantsprayzusammensetzung, eine Tonersprayzusammensetzung, eine feuchtigkeitsspendende Sprayzusammensetzung, eine Desinfektionssprayzusammensetzung, eine schwellungslindernde Sprayzusammensetzung, eine Wundbehandlungssprayzusammensetzung, eine Antiallergensprayzusammensetzung, eine Narbenbehandlungszusammensetzung, eine antimikotische Sprayzusammensetzung und eine antibakterielle und/oder antivirale dermatologische Sprayzusammensetzung ist.

3. Verwendung nach einem der vorhergehenden Ansprüche, wobei die durchschnittliche Länge der MFC-Fibrillen 100 nm bis 50 µm, bevorzugt 500 nm bis 25 µm, bevorzugter 1 µm bis 10 µm, am bevorzugtesten 3 µm bis 10 µm beträgt.

4. Verwendung nach einem der vorhergehenden Ansprüche, wobei der durchschnittliche Durchmesser der MFC-Fibrillen 1 nm bis 500 nm, bevorzugt 5 nm bis 100 nm, bevorzugter 10 nm bis 50 nm, am bevorzugtesten 10 nm bis 30 nm beträgt.

5. Verwendung nach einem der vorhergehenden Ansprüche, wobei die mikrofibrillierte Cellulose zu einer Dispersion mit einer Nullscherviskosität, η₀, von mindestens 5000 Pa*s, bevorzugt mindestens 6000 Pa*s, bevorzugter mindestens 7000 Pa*s führt, bestimmt in Polyethylenglykol (PEG) als Lösungsmittel und bei einem Feststoffgehalt der MFC von 0,65% bei einer Temperatur von 25°C.

6. Verwendung nach einem der vorhergehenden Ansprüche, wobei die Wasserhaltekapazität der MFC mindestens 75, bevorzugt mindestens 80, bevorzugter mindestens 100 beträgt.

7. Verwendung nach einem der vorhergehenden Ansprüche, wobei die MFC durch physikalische Adsorption von mindestens einer amphiphilen Verbindung, die eine nichtionische amphiphile Verbindung ist, physikalisch modifiziert ist.

8. Verwendung nach einem der vorhergehenden Ansprüche, wobei die MFC im Wesentlichen frei von kationischen Substituenten ist.

9. Verwendung nach einem der vorhergehenden Ansprüche, wobei der Trockengehalt der MFC 0,1% bis 100%, bevorzugt 0,2% bis 40%, bevorzugter 0,5% bis 25%, bevorzugter 1% bis 20% beträgt, bevor die MFC zu der Zusammensetzung hinzugefügt wird.

10. Verwendung nach einem der vorhergehenden Ansprüche, wobei die Zusammensetzung ferner mindestens einen UV-Filter umfasst.

## Revendications

1. Utilisation de cellulose micro-fibrillée (MFC) comme un agent anti-coulant dans une composition de soin de la peau pulvérisable,
dans laquelle le MFC est un MFC non modifié ou physiquement modifié par adsorption physique de au moins un composé amphiphile,
dans laquelle la concentration du MFC dans la composition de soin de la peau pulvérisable est de 0.012% à 0.6% en poids, et
dans laquelle le MFC provient de bois, plantes annuelles, coton, lin, paille, ramie, bagasse de canne à sucre, certaines algues, jute, betterave sucrière, agrumes, déchets de l'industrie alimentaire ou de cultures énergétiques.

2. Utilisation selon la revendication 1, dans laquelle la composition de soin de la peau pulvérisable est une composition d'écran solaire en spray, une composition d'après-soleil en spray, une composition pour le visage en spray, une composition pour le corps en spray, une composition de répulsif anti-insectes en spray, une composition pour le bronzage en spray, une composition de déodorant en spray, une composition de tonique en spray, une composition hydratante en spray, une composition désinfectante en spray, une composition de soulagement de l'oedème en spray, une composition de soin des plaies en spray, une composition anti-allergène en spray, une composition de traitement de cicatrice, une composition antimycosique en spray et une composition dermatologique antibactérienne et/ou antivirale en spray.

3. Utilisation selon l'une quelconque des revendications précédentes, dans laquelle la longueur moyenne de fibrille du MFC est de 100 nm à 50 µm, préférentiellement de 500 nm à 25 µm, plus préférentiellement de 1 µm à 10 µm, encore plus préférentiellement de 3 µm à 10 µm.

4. Utilisation selon l'une quelconque des revendications précédentes, dans laquelle le diamètre moyen de fibrille du MFC est de 1 nm à 500 nm, préférentiellement de 5 nm à 100 nm, plus préférentiellement de 10 nm à 50 nm, encore plus préférentiellement de 10 nm à 30 nm.

5. Utilisation selon l'une quelconque des revendications précédentes, dans laquelle la cellulose micro-fibrillée entraine une dispersion ayant une viscosité à cisaillement nul, η₀, de au moins 5000 Pa*s, préférentiellement au moins 6000 Pa*s, plus préférentiellement au moins 7000 Pa*s, telle que mesurée dans du polyéthylène glycol (PEG) comme solvant, et à une teneur en solides de MFC de 0.65%, à une température de 25°C.

6. Utilisation selon l'une quelconque des revendications précédentes, dans laquelle la capacité de rétention de l'eau du MFC est de au moins 75, préférentiellement au moins 80, plus préférentiellement au moins 100.

7. Utilisation selon l'une quelconque des revendications précédentes, dans laquelle le MFC est physiquement modifié par adsorption physique de au moins un composé amphiphile qui est un composé amphiphile non ionique.

8. Utilisation selon l'une quelconque des revendications précédentes, dans laquelle le MFC est sensiblement libre de substituants cationiques.

9. Utilisation selon l'une quelconque des revendications précédentes, dans laquelle la teneur en matière sèche du MFC est de 0.1% à 100%, préférentiellement de 0.2% à 40%, plus préférentiellement de 0.5% à 25%, plus préférentiellement de 1% à 20%, avant que le MFC ne soit ajouté à la composition.

10. Utilisation selon l'une quelconque des revendications précédentes, dans laquelle la composition comprend en outre au moins un filtre UV.
